# EUROPÄISCHE PATENTSCHRIFT

(11) **EP 0 906 302 B1**
(45) Veröffentlichungstag und Bekanntmachung des Hinweises auf die Patenterteilung: **08.08.2001**
(21) Anmeldenummer: 97922985.3
(22) Anmeldetag: 07.05.1997
(51) Int. Cl.: C07D 311/66, C07B 57/00

(54) **ENANTIOMERENTRENNUNG VON CHROMANSÄUREESTERN**
ENANTIOMER SEPARATION FROM CHROMANE ACID ESTERS
SEPARATION D'ENANTIOMERES CONTENUS DANS DES ESTERS D'ACIDE CHROMANIQUE

(30) Priorität: 14.06.1996 DE 19623755
(43) Veröffentlichungstag der Anmeldung: 07.04.1999
(73) Patentinhaber: MERCK PATENT GmbH, 64293 Darmstadt (DE)
(72) Erfinder: DEVANT, Ralf, M., D-64291 Darmstadt (DE); REUBOLD, Helmut, D-64732 Bad König (DE); SCHULTE, Michael, D-65428 Rüsselsheim (DE)
(86) Internationale Anmeldenummer: EP9702314
(87) Internationale Veröffentlichungsnummer: WO9747617

(56) Entgegenhaltungen:
- JOURNAL OF MEDICINAL CHEMISTRY, Bd. 38, 1995, WASHINGTON US, Seiten 858-868, XP002037316 TH.D. PENNING ET AL.: "SECOND GENERATION LEUKOTRIENE B4 ANTAGONISTS RELATED TO SC 41930." in der Anmeldung erwähnt

## Beschreibung

Die Erfindung betrifft die chromatographische Enantiomerentrennung von Estern von 2-Alkanoyl-chromanderivaten der Formel I, insbesondere mittels kontinuierlichen Verfahren.

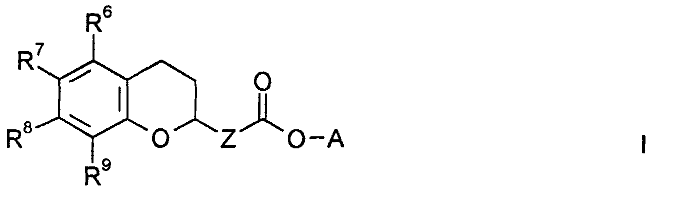

In Formel I bedeuten:
- Z: -(CH₂)ₙ₁-(CHA)ₙ₂-(CH₂)ₙ₃, wobei
n1 = 0, 1, 2 oder 3;
n2= 0 oder 1;
n3 = 0, 1, 2 oder 3 mit der Maßgabe, daß
n1 + n2+ n3 < 4,
- R⁶, R⁷, R⁸ und R⁹: unabhängig voneinander H, A, OA, Phenoxy, OH, F, Cl, Br, I, CN, CF₃, NO₂, NH₂, NHA, NA₂, Ac, Ph, Cycloalkyl mit 3-7 C-Atomen, -CH₂NH₂, -CH₂NHA, -CH₂NA₂, -CH₂NHAc oder -CH₂NHSO₂CH₃, wobei auch zwei benachbarte Reste eine Alkylenkette mit 3 oder 4 C-Atomen bedeuten können,
- A: Alkyl mit 1-6 C-Atomen,
- Ac: Alkanoyl mit 1-10 C-Atomen or Aroyl mit 7-11 C-Atomen,
- Ph: unsubstituiertes oder mit R⁵, 2-, 3- oder 4-Pyridyl oder Phenoxy substituiertes Phenyl,
- R⁵: unsubstituiertes oder mit 1-5 F, oder mit CF₃, oder mit ganz oder teilweise fluorsubstituiertem A, A, und/oder OA substituiertes Phenyl.

Ester der 2-Alkanoyl-chromanderivate nach Formel I, insbesondere solche, bei denen die Summe n1 + n2 + n3 den Wert 0 oder 1 aufweist (im letzteren Fall bedeutet Z bevorzugt -CH₂-), sind wichtige Zwischenprodukte insbesondere bei der Herstellung von pharmazeutisch wirksamen Amino(thio)etherderivaten, wie sie beispielsweise in EP-A-0 707 007 offenbart sind. Soweit diese Chromanderivate optisch aktiv sind, ist die Trennung der Enantiomeren notwendig oder wünschenswert.

Für bestimmte Chromanderivate sind in J. Heterocyclic Chem. 29, 431 ff (1992) Synthesewege für enantiomerenreine Chromanderivate beschrieben. Diese Synthesen sind jedoch nicht ohne weiteres auf andere ähnliche Verbindungen übertragbar. In J.Med.Chem. **38**, Seiten 858 - 868 (1995) wird im Zusammenhang mit Leukotrien B₄-Rezeptorantagonisten vorgeschlagen, Enantiomere bestimmter Benzopyrancarbonsäurederivate durch batch-Chromatographie an chiralen Phasen zu trennen. Weiterhin wird für die Enantiomerentrennung von Chroman-2-carboxylaten in EP-A-0 448 254 vorgeschlagen, mittels mikrobieller Lipase einen der enantiomeren Ester zu verseifen. Dieses Verfahren ist nur anwendbar, soweit der vorgelegte Chromanester durch die Lipase spaltbar ist.

Es stellt sich somit die Aufgabe, Enantiomere von derivatisierten Chromanestern zu trennen, ohne durch die limitierte Substrat-spezifität eingeschränkt zu sein.

Grundsätzlich lassen sich Enantiomere an chiralen Sorbentien trennen. Dem Fachmann sind eine große Anzahl chiraler Sorbentien, beispielsweise solche auf der Grundlage von Cellulosederivaten, Cyclodextrinen, oder Poly(meth)acrylamidderivaten mit optischaktiver Seitenkette bekannt. Solche chiralen Sorbentien und deren Verwendung sind beispielsweise in EP-A-0 147 804,
EP-A-0 155 637, DE 36 19 303, DE 40 05 868 oder DE 40 06 923 offenbart.

Es wurde gefunden, daß die Enantiomerentrennung von Chroman-2-carbonsäureestern, insbesondere von Chroman-2-carbonsäureethylester an einer Reihe üblicher chiraler Sorbentien nicht möglich war: Polymethacrylamidderivate gebunden an Kieselgel (DE 36 19 303; ChiraSpher®; Fa. Merck, DE), Cyclodextrinderivate gebunden an Kieselgel (DE 40 06 923; ChiraDex®; Fa. Merck, DE), Dinitrobenzoyl-Phenylglycin (Fa. Baker) erwiesen sich als ungeeignet. Auch unter Verwendung zweier Sorbentien auf Cellulosebasis war keine Trennung möglich: Weder auf Cellulosetriacetat (Fa. Merck, DE) noch auf Cellulose-tris-(3,5-dimethylbenzoat) (adsorbiert auf Kieselgel; CHIRALCEL® OJ; Fa. Daicel, JP) waren die Enantiomeren zu trennen. Überraschend wurde jedoch gefunden, daß eine Trennung auf einem Sorbens auf Cellulosebasis, das dem CHIRALCEL® OJ ähnelt, und bei dem derselbe aromatische Substituent (3,5-Dimethylphenyl) über eine Carbamatbindung anstelle einer Esterbindung gebunden vorliegt, möglich ist: Die Trennung an Cellulose-tris-(3,5-dimethylphenylcarbamat) (adsorbiert auf Kieselgel; CHIRALCEL® OD; Fa. Daicel, JP) war mit guten Trennfaktoren mit Alkoholen oder Alkohol-Kohlenwasserstoff-Mischungen als Elutionsmittel sowohl mittels Säulenchromatographie (batch-Verfahren) als auch mittels der kontinuierlichen "simulated moving bed"- Chromatographie (SMB-Chromatographie) möglich. In ähnlicher Weise war auch die Trennung an einem Sorbens, bei dem die 3,5-Dimethylphenylcarbamatreste an Amylose gebunden sind, möglich (CHIRALPAK® AD; Fa. Daicel, JP). Als gute Sorbentien erwiesen sich im übrigen auch nach EP 0 316 270 hergestellte poröse Celluloseester, z.B. Cellulosetribenzoat.

Als besonders geeignete Elutionsmittel erwiesen sich C₁ bis C₅-Alkohole, insbesondere Methanol und Ethanol, oder deren Mischungen, sowie Mischungen aus C₁ bis C₅-Alkoholen und C₅ bis C₁₀-Kohlenwasserstoffen, insbesondere Mischungen aus Hexan oder Heptan mit 2-Propanol.

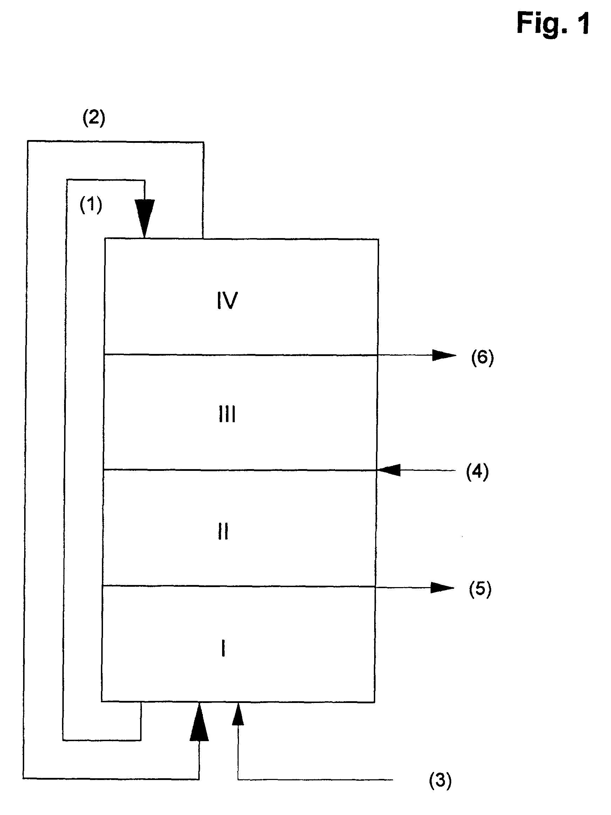

In Abbildung 1 ist das Verfahren der Gegenstromchromatographie, das die Grundlage der "simulated moving bed"-Chromatographie (SMB-Chromatographie) darstellt, schematisch dargestellt. Darin bedeutet (1) den Strom des Sorbens. Im SMB-Verfahren wird der physikalisch nur schwer zu realisierende Strom des Sorbens simuliert durch cyclisches Umschalten von Mehrwegeventilen, welche mehrer zu einem Kreislauf geschaltete Säulen verbinden.

Die experimentelle Realisierung der Trennung wurd auf einer SMB-Anlage ausgeführt, die nach dem nachfolgend erläuterten Vier-Zonen-Modell arbeitet. Erfindungsgeäß können auch SMB-Anlagen verwendet werden, die nach anderen Modellen, z.B. dem Drei-Zonen-Modell arbeiten. Geeignete Verfahrensvarianten sind dem Fachmann aus der Literatur bekannt.

Gegenstand der Erfindung sind Verfahren zur Enantiomerentrennung von derivatisierten Chromanestern der Formel I, insbesondere von Chroman-2-carbonsäureestern, insbesondere wiederum von Chroman-2-carbonsäureethylester, mittels Chromatographie an Sorbentien, die mit aromatischen Carbamaten substituierte Polysaccharide, beispielsweise Cellulose, insbesondere Cellulose-tris-(3,5-dimethylphenylcarbamat), enthalten, unter Verwendung von Elutionsmitteln mit einem Gehalt an C₁ bis C₅-Alkoholen. Als Elutionsmittel sind C₁ bis C₅-Alkohole, insbesondere Methanol und Ethanol, oder deren Mischungen geeignet. Bevorzugte Elutionsmittel sind Mischungen aus C₁ bis C₅-Alkoholen und C₅ bis C₁₀-Kohlenwasserstoffen, insbesondere Mischungen aus Hexan oder Heptan mit 2-Propanol.

Derivatisierte Chromanester der Formel I, sowie die bevorzugten Bedeutungen der in Formel I genannten Reste sind in EP-A-0 707 007 offenbart. Bevorzugte Verbindungen der Formel I sind die Chroman-2-carbonsäureester, insbesondere der Chroman-2-carbonsäureethylester. Für die bevorzugten derivatisierten Chromanester besitzen die Reste aus Formel I die folgenden Bedeutungen: R⁶, R⁷, R⁸ und R⁹ bedeuten H; in Z sind n1, n2 und n3 gleich 0; insbesondere bevorzugt ist der Ethylester (A gleich Ethyl).

Die als Elutionsmittel genannten C₁ bis C₅-Alkohole bedeuten erfindungsgemäß Methanol, Ethanol, n-Propanol, i-Propanol, n-Butanol, i-Butanol; bevorzugt werden Methanol oder Ethanol. Auch Mischungen dieser Alkohole können erfindungsgemäß verwendet werden. Besonders bevorzugt als Elutionsmittel sind erfindungsgemäß Mischungen aus den bereits genannten C₁ bis C₅-Alkoholen und linearen, verzweigten oder cyclischen C₅ bis C₁₀-Kohlenwasserstoffen, wobei die Mischungen aus mehr als einem der genannten Alkohole und mehr als einem dieser Kohlenwasserstoffe bestehen können. Beispielhaft für die linearen, verzweigten oder cyclischen C₅- bis C₁₀-Kohlenwasserstoffe seien genannt: n-Pentan, Isopentan, n-Hexan, Isohexan, Cyclohexan, n-Heptan, Isoheptan, n-Octan, Isooctan. In den Mischungen aus Alkohol und Kohlenwasserstoff beträgt der Anteil an Kohlenwasserstoff bevorzugterweise zwischen 70 und 99 Volumen-%, besonders bevorzugterweise zwischen 85 und 95 Volumen-%.

Die erfindungsgemäße Trennung kann im konventionellen Batch-Verfahren ausgeführt werden. Bevorzugt ist die Trennung mittels des kontinuierlich arbeitenden SMB-Verfahrens, wie es im folgenden bezugnehmend auf Abbildung 1 näher erläutert wird.

Die Voraussetzung für eine Enantiomerentrennung in präparativem Maßstab ist eine möglichst gute Trennung (Basislinientrennung, hoher Selektivitätsfaktor α). Da zudem bei der üblichen batchweisen Chromatographie zu einem bestimmten Zeitpunkt der Trennung nur der Bereich der Trennsäule genutzt wird in dem sich das zu trennende Material gerade auf seinem Weg durch die Säule befindet, benötigt man sehr leistungsfähige Trennsäulen (hohe Anzahl theoretischer Böden). Insgesamt ist bei der konventionellen Säulentrennung insbesondere die Zeit-Volumen-Leistung nicht sehr hoch; entsprechend kostenintensiv sind derartige Verfahren. Beim Einsatz von kontinuierlichen Verfahren, beispielsweise der SMB-Chromatographie wird eine erheblich verbesserte Zeit-Volumen-Leistung erreicht. Bei der SMB-Chromatographie handelt es sich um ein kontinuierliches Gegenstrom - Verfahren, bei dem die mobile und die stationäre Phase in entgegengesetzte Richtungen geführt werden (Chirality 5, 267 ff. (1993)). Dadurch wird, anders als beim batch - weisen Vorgehen, zu jedem Zeitpunkt einer Trennung die gesamte stationäre Phase genutzt, was die Selektivität des Trennsystems deutlich erhöht. Verglichen mit der Batch-Chromatographie benötigt man also bei der SMB eine erheblich geringere Anzahl theoretischer Böden.

Durch das Gegenstromprinzip ist die SMB für die Auftrennung von Zweistoffgemischen (z.B. die beiden Enantiomere eines Racemates) in idealer Weise geeignet.

Die kontinuierliche Arbeitsweise des SMB Verfahrens, wie es beispielhaft in Abbildung 1 schematisch dargestellt ist, erlaubt die Einstellung eines zeitlich stationären Zustandes bei dem kontinuierlich Eluent (3), sowie eine Lösung des zu trennenden Zweistoffgemisches (Feed; (4)) dem System zugeführt und ebenso kontinuierlich die beiden getrennten Komponenten (Raffinat (6) und Extrakt (5)) aus dem System herausgeführt werden können. Das Zu- und Herausführen der genannten Stoffströme erfolgt mit Hilfe von 4 Pumpen (nicht dargestellt). Der Hauptstrom des Eluenten (2) wird mit einer weiteren Pumpe im Kreislauf geführt (Recycling-Pumpe; nicht dargestellt). Da deshalb dem System nur eine geringere Menge an frischem Eluenten zugeführt werden muß (Feed + Eluent₍ₙₑᵤ₎ = Raffinat + Extrakt), ist der Lösungsmittelverbrauch pro Produkteinheit bei der SMB deutlich geringer als im Falle der Batch-Chromatographie. Das Säulenbett einer stationären Phase unterteilt sich bei der SMB in 4 Zonen (je eine Adsoprtions- und Desorptionszone für die beiden zu trennenden Komponenten), welche relativ zu den Zufuhr- und Auslaßpunkten definiert sind:

| | |
|---|---|
| Zone I | - zwischen Eluent- und Extrakt-Leitung |
| Zone II | - zwischen Extrakt- und Feed-Leitung |
| Zone III | - zwischen Feed- und Raffinat-Leitung |
| Zone IV | - zwischen Raffinat und Eluent-Leitung |

Im Falle der Trennung von Zweistoffgemischen lassen sich nun Bedingungen, d.h. Flußraten in den Zonen I-IV, finden, bei denen sich die schwächer retinierte Komponente mit der mobilen Phase und die stärker retinierte Komponente mit der stationären Phase bewegt. Die getrennten Komponenten können dann in reiner Form mit dem Extrakt- beziehungweise Raffinat-Strom entnommen werden.

Es ist technisch nur sehr schwer möglich, eine tatsächliche Bewegung einer stationären Phase (1) zu realisieren. Deshalb wird diese Bewegung der stationären Phase simuliert. Dazu wird das gesamte Säulenbett in zyklisch hintereinandergeschaltete Einzelsäulen unterteilt. Die Gesamtzahl der Säulen ist ein Vielfaches der Zahl 4, da das System, wie oben erwähnt, 4 chromatographische Zonen besitzt. Zwischen den Einzelsäulen befinden sich je 4 Zweiwegeventile, die eine Verbindung zu den 4 Zufuhr- und Auslaßleitungen darstellen. Aufgrund dieser Ventile, kann also jeder Punkt zwischen den Säulen jede Funktion (Eluent-, Feed-Zufuhr oder Raffinat- bez. Extrakt-Auslaß) einnehmen. Zu einem gegebenen Zeitpunkt definiert die Lage der 4 Zufuhr- und Auslaß-Leitungen die 4 chromatographischen Zonen. Wird nun die Position der 4 Leitungen nach einer definierten Zeit um eine Säuleneinheit in Richtung der Fließmittelbewegung weitergeschaltet, so entspricht dies einer Bewegung des Säulenbettes in die entgegengesetzte Richtung. Durch Weiterschaltung der Speisepunkte in definierten Zeitabständen durchläuft damit jede Einzelsäule nacheinander alle 4 Zonen, bis die Zufuhr- und Auslaß-Leitungen wieder ihre ursprüngliche Position einnehmen und somit ein Zyklus abgeschlossen ist.

Nachdem mehrere Zyklen durchlaufen wurden, stellt sich ein stationärer Zustand ein, der es bei geeigneter Wahl der Fließgeschwindigkeiten im System und geeigneter Taktzeit für die Ventilschaltungen ermöglicht, die getrennten Produkte in reiner Form als Extrakt- und Raffinatströme abzunehmen.

Auch ohne weitere Ausführungen wird davon ausgegangen, daß ein Fachmann die obige Beschreibung in weitesten Umfang nutzen kann. Die bevorzugten Ausführungsformen sind deswegen lediglich als beschreibende, keineswegs als in irgendeine Weise limitierende Offenbarung aufzufassen.

Die vollständige Offenbarung aller vor- und nachstehend aufgeführten Anmeldungen, Patente und Veröffentlichungen, sowie der korrespondierenden Anmeldung DE 196 23 755.6, eingereicht am 14.06.96, sind durch Bezugnahme in diese Anmeldung eingeführt.

### Beispiele:

Die folgenden Beispiele sollen die Erfindung verdeutlichen; sie bedeuten keine Einschränkung des Erfindungsgedankens. Beispielhaft werden verschiedene Varianten der erfindungsgemäßen Enantiomerentrennung unter Verwendung von Chroman-2-carbonsäureethylester beschrieben.

Soweit als Elutionsmittel Gemische angegeben werden, so erfolgen die Angaben in Volumenverhältnissen (v:v).

### Beispiel 1: Enantiomerentrennung von Chroman-2-carbonsäureethylester an CHIRALCEL® OD (Methanol als Elutionsmittel)

Experimentelle Bedingungen:

| | |
|---|---|
| Säule | CHIRALCEL® OD (Daicel; Säulendimensionen: 250*4 mm) |
| Elutionsmittel | Methanol |
| Flußrate | 0,8 ml/min |
| Detektion | UV bei 220 nm |

### Ergebnis: Das erste Enantiomer wird nach 5,35 Minuten, das zweite nach 6,73 Minuten eluiert (α = 1,95).

### Beispiel 2: Enantiomerentrennung von Chroman-2-carbonsäureethylester an CHIRALCEL® OD (Hexan/2-Propanol (90:10; v:v) als Elutionsmittel)

Experimentelle Bedingungen wie Beispiel 1; jedoch Hexan/ 2-Propanol (90:10; v:v) als Elutionsmittel.

### Ergebnis: Das erste Enantiomer wird nach 8,19 Minuten, das zweite nach 43,63 Minuten eluiert (α = 9,26).

### Beispiel 3: Enantiomerentrennung von Chroman-2-carbonsäureethylester an CHIRALCEL® OD (weitere Elutionsmittel)

Experimentelle Bedingungen wie Beispiel 1; jedoch a) Ethanol und b) Heptan/2-Propanol (90:10; v:v) als Elutionsmittel.

### Ergebnisse:

| Fließmittel: | Retentionszeit (1. Enantiomer) | Retentionszeit (2. Enantiomer) | α |
|---|---|---|---|
| (a)Ethanol | 5,17 min | 7,27 min | 2,65 |
| (b)Heptan/2-Propanol (90:10) | 9,27 min | 44,32 min | 7,53 |

### Beispiel 4: Enantiomerentrennung von Chroman-2-carbonsäureethylester an CHIRALPAK® AD (Ethanol als Elutionsmittel)

Experimentelle Bedingungen:

| | |
|---|---|
| Säule | CHIRALPAK® AD (Daicel; Säulendimensionen: 250*4 mm) |
| Elutionsmittel | Ethanol |
| Flußrate | 0,8 ml/min |
| Detektion | UV bei 220 nm |

### Ergebnis: Das erste Enantiomer wird nach 5,19 Minuten, das zweite nach 6,29 Minuten eluiert (α = 2,01).

### Beispiel 5: Enantiomerentrennung von Chroman-2-carbonsäureethylester an CHIRALPAK® AD (Methanol als Elutionsmittel)

Experimentelle Bedingungen wie Beispiel 4; jedoch Methanol als Elutionsmittel.

### Ergebnis: Das erste Enantiomer wird nach 5,27 Minuten, das zweite nach 7,08 Minuten eluiert (α = 2,55).

### Beispiel 6: Enantiomerentrennung von Chroman-2-carbonsäureethylester an porösem Cellulosetribenzoat

Experimentelle Bedingungen:

| | |
|---|---|
| Säule | Poröses beadförmiges Cellulosetribenzoat (Säulendimensionen: 3 Säulen je 125*4 mm) |
| Elutionsmittel | Methanol |
| Flußrate | 0,5 ml/min |
| Detektion | UV bei 220 nm |

### Ergebnis: Das erste Enantiomer wird nach 17,50 Minuten, das zweite nach 23,10 Minuten eluiert (α = 1,53).

### Beispiel 7: Enantiomerentrennung von Chroman-2-carbonsäureethylester an CHIRALCEL® OD (präparative Säulentrennung im batch-Verfahren)

| | |
|---|---|
| Stationäre Phase | Chiralcel® OD (Korngröße: 20 µm) |
| Säulendimensionen | 250*50 mm |
| Flußrate | 45 ml/min |
| Fließmittel | Heptan/2-Propanol (90:10; v:v) |

Bei einer Aufgabemenge von 1 ml Chroman-2-carbonsäureester (racemische Reinsubstanz) ergibt sich noch eine Basislinientrennung; d.h. die Reinheit der getrennten Enantiomere ist > 99,5 %.

### Beispiel 8: Enantiomerentrennung von Chroman-2-carbonsäureethylester an CHIRALCEL® OD (präparative Trennung im kontinuierlichen SMB-Verfahren)

| | |
|---|---|
| SMB-System | LICOSEP® 12-26 (Separex), bestückt mit 8 Superformance® Säulen (Merck; 26 mm Innendurchmesser) |
| Stationäre Phase | CHIRALCEL® OD (Korngröße: 20 µm; Länge des Säulenbettes: 100 mm) |
| Fließmittel | Heptan/2-Propanol (90:10) |
| Temperatur | 25 °C |
| Feed-Konzentration | 10 g/l |
| Feed Flußrate | 5 ml/min |
| Recycling Flußrate | 50 ml/min |

Mit diesen Parameter wird ein Durchsatz von 200 g Racemat pro 24 h erreicht. die Reinheiten von Raffinat und Extrakt liegen bei 99 %.

### Vergleichsbeispiel A: Enantiomerentrennung von Chroman-2-carbonsäureethylester an Cellulosetriacetat

Experimentelle Bedingungen:

| | |
|---|---|
| Säule | Cellulosetriacetat (Merck; Säulendimensionen: 250*10 mm) |
| Elutionsmittel | Methanol |
| Flußrate | 1,8 ml/min |
| Detektion | UV bei 220 nm |

### Ergebnis: Die beiden Enantiomere eluieren ungetrennt nach 15,6 Minuten (α = 0,00).

### Vergleichsbeispiel B: Enantiomerentrennung von Chroman-2-carbonsäureethylester an Cellulose-tris(3,5-dimethylbenzoat) (Methanol als Elutionsmittel)

Experimentelle Bedingungen:

| | |
|---|---|
| Säule | Cellulose-tris-(3,5-dimethylbenzoat), adsorbiert an Kieselgel (CHIRALCEL® OJ: Daicel; Säulen-dimension: 250*4 mm) |
| Elutionsmittel | Methanol |
| Flußrate | 0,8 ml/min |
| Detektion | UV bei 220 nm |

### Ergebnis: Beide Enantiomere eluieren ungetrennt nach 11,50 Minuten (α = 0,00).

### Vergleichsbeispiel C: Enantiomerentrennung von Chroman-2-carbonsäureethylester an Cellulose-tris(3,5-dimethylbenzoat) (Ethanol als Elutionsmittel)

Experimentelle Bedingungen wie Vergleichsbeispiel B; jedoch Ethanol als Elutionsmittel.

Ergebnis: Die Enantiomeren eluieren nach 10,8 beziehungsweise 11,1 Minuten (α = 1,04), die Elutionspeaks überlappen sich jedoch fast vollständig.

## Patentansprüche

1. Verfahren zur chromatographischen Trennung von enantiomeren Estern von 2-Alkanoyl-chromanderivaten der Formel I, 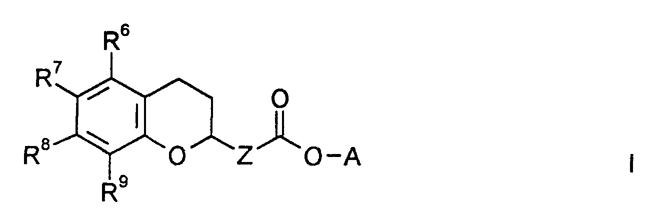 worin
Z -(CH₂)ₙ₁-(CHA)ₙ₂-(CH₂)ₙ₃ wobei
n1 = 0, 1, 2 oder 3;
n2= 0 oder 1;
n3 = 0, 1, 2 oder 3 mit der Maßgabe, daß
n1 + n2+ n3 < 4,
R⁶, R⁷, R⁸ und R⁹ unabhängig voneinander H, A, OA, Phenoxy, OH, F, Cl, Br, I, CN, CF₃, NO₂, NH₂, NHA, NA₂, Ac, Ph, Cycloalkyl mit 3-7 C-Atomen, -CH₂NH₂, -CH₂NHA, -CH₂NA₂,-CH₂NHAc oder -CH₂NHSO₂CH₃, wobei auch zwei benachbarte Reste eine Alkylenkette mit 3 oder 4 C-Atomen bedeuten können,
A Alkyl mit 1-6 C-Atomen,
Ac Alkanoyl mit 1-10 C-Atomen oder Aroyl mit 7-11 C-Atomen,
Ph unsubstituiertes oder mit R⁵, 2-, 3- or 4-Pyridyl oder Phenoxy substituiertes Phenyl
R⁵ unsubstituiertes oder mit 1-5 F, oder mit CF₃, oder mit ganz oder teilweise fluorsubstituiertem A, A, und/oder OA substituiertes Phenyl.
bedeuten,
dadurch gekennzeichnet, daß die Trennung an einem Sorbens erfolgt, das mit aromatischen Carbamaten substituierte Polysaccharide enthält, und daß ein Elutionsmittel mit einem Gehalt an C₁- bis C₅-Alkoholen verwendet wird.

2. Verfahren nach Anspruch 1, dadurch gekennzeichnet, daß als substituiertes Polysaccharid ein Cellulosederivat im Sorbens enthalten ist.

3. Verfahren nach Anspruch 1 oder 2, dadurch gekennzeichnet, daß Enantiomere getrennt werden, wobei die in Formel I genannten Reste die folgende Bedeutung besitzen: R⁶, R⁷, R⁸ und R⁹ bedeuten H; in Z sind n1, n2 und n3 gleich 0.

4. Verfahren nach Anspruch 3, dadurch gekennzeichnet, daß Enantiomere getrennt werden, wobei der Rest A in Formel I Ethyl bedeutet.

5. Verfahren nach einem der Ansprüche 1-4, dadurch gekennzeichnet, daß das Sorbens Cellulose-tris-(3,5-dimethylphenylcarbamat) enthält.

6. Verfahren nach einem der Ansprüche 1-5, dadurch gekennzeichnet, daß als Elutionsmittel ein C₁ bis C₅-Alkohol verwendet wird.

7. Verfahren nach einem der Ansprüche 1-5, dadurch gekennzeichnet, daß als Elutionsmittel eine Mischung enthaltend einen C₁ bis C₅-Alkohol und einen C₅ bis C₁₀-Kohlenwasserstoff verwendet wird.

8. Verfahren nach einem der Ansprüche 1-7, dadurch gekennzeichnet, daß das Verfahren im batch-Verfahren ausgeführt wird.

9. Verfahren nach einem der Ansprüche 1-7, dadurch gekennzeichnet, daß das Verfahren kontinuierlich nach dem SMB-Verfahren ausgeführt wird.

## Claims

1. Process for the chromatographic resolution of enantiomeric esters of 2-alkanoylchroman derivatives of the formula I, 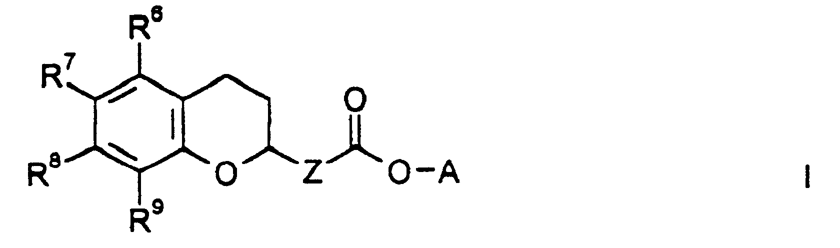 in which
Z is -(CH₂)ₙ₁-(CHA)ₙ₂-(CH₂)ₙ₃, where
n1 = 0, 1, 2 or 3;
n2 = 0 or 1;
n3 = 0, 1, 2 or 3 with the proviso that
n1 + n2 + n3 < 4,
R⁶,R⁷,R⁸ and R⁹ independently of one another are H, A, OA, phenoxy, OH, F, Cl, Br, I, CN, CF₃, NO₂, NH₂, NHA, NA₂, Ac, Ph, cycloalkyl having 3-7 C atoms, -CH₂NH₂, -CH₂NHA, -CH₂NA₂, -CH₂NHAc or -CH₂NHSO₂CH₃, where two adjacent radicals can also be an alkylene chain having 3 or 4 C atoms,
A is alkyl having 1-6 C atoms,
Ac is alkanoyl having 1-10 C atoms or aroyl having 7-11 C atoms,
Ph is phenyl which is unsubstituted or substituted by R⁵, 2-, 3- or 4-pyridyl or phenoxy,
R⁵ is phenyl which is unsubstituted or substituted by 1-5 F, or by CF₃, or by completely or partially fluorine-substituted A and/or OA,
characterized in that the resolution is carried out on a sorbent which contains polysaccharides substituted by aromatic carbamates, and in that an eluent containing C₁ to C₅-alcohols is used.

2. Process according to Claim 1, characterized in that the substituted polysaccharide contained in the sorbent is a cellulose derivative.

3. Process according to Claim 1 or 2, characterized in that enantiomers are resolved in which the radicals mentioned in formula I have the following meaning: R⁶, R⁷, R⁸ and R⁹ are H; in Z n1, n2 and n3 are equal to 0.

4. Process according to Claim 3, characterized in that enantiomers are resolved in which the radical A in formula I is ethyl.

5. Process according to one of Claims 1 - 4, characterized in that the sorbent contains cellulose tris(3,5-dimethylphenylcarbamate).

6. Process according to one of Claims 1 - 5, characterized in that the eluent used is a C₁ to C₅-alcohol.

7. Process according to one of Claims 1 - 5, characterized in that the eluent used is a mixture comprising a C₁ to C₅-alcohol and a C₅ to C₁₀-hydrocarbon.

8. Process according to one of Claims 1 - 7, characterized in that the process is carried out in the batch process.

9. Process according to one of Claims 1 - 7, characterized in that the process is carried out continuously according to the SMB process.

## Revendications

1. Procédé pour la séparation chromatographique d'esters énantiomères de dérivés de 2-alcanoyl-chromanne de formule I 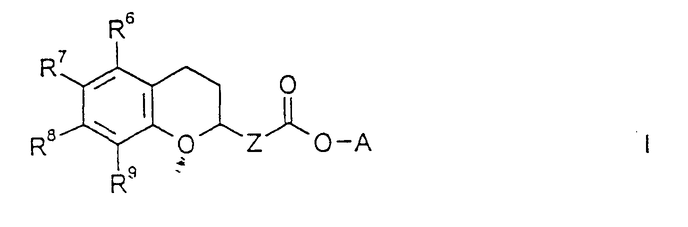 dans laquelle
Z est -(CH₂)ₙ₁-(CHA)ₙ₂-(CH₂)ₙ₃, où n1 = 0, 1, 2 ou 3 ; n2 = 0 ou 1 ; n3 = 0, 1, 2 ou 3, à la condition que nl + n2 + n3 < 4,
R⁶, R⁷, R⁸ et R⁹ représentent chacun indépendamment des autres H, A, OA, le groupe phénoxy, OH, F, Cl, Br, I, CN, CF₃, NO₂, NH₂, NHA, NA₂, Ac, Ph, un groupe cycloalkyle ayant de 3 à 7 atomes de carbone, -CH₂NH₂, -CH₂NHA, -CH₂NA₂, -CH₂NHAc ou -CH₂NHSO₂CH₃, où deux radicaux voisins peuvent aussi représenter une chaîne alkylène ayant 3 ou 4 atomes de carbone,
A est un groupe alkyle ayant de 1-6 atomes de carbone,
Ac est un groupe alcanoyle ayant de 1 à 10 atomes de carbone ou aroyle ayant de 7 à 11 atomes de carbone,
Ph est un groupe phényle non-substitué, ou substitué par R⁵, un groupe 2-, 3- ou 4-pyridyle, ou phénoxy,
R⁵ est un groupe phényle non-substitué ou substitué par 1 à 5 atomes de fluor, ou par CF₃, ou par le radical A entièrement ou partiellement fluoré, A, et/ou OA,
caractérisé en ce que la séparation s'effectue sur un sorbant qui contient des polysaccharides substitués par des carbamates aromatiques, et qu'on utilise un éluant contenant des alcools en C₁ à C₅.

2. Procédé selon la revendication 1, caractérisé en ce que le sorbant contient en tant que polysaccharide substitué un dérivé de la cellulose.

3. Procédé selon la revendication 1 ou 2, caractérisé par la séparation d'énantiomères, les radicaux mentionnés dans la formule I ayant les significations suivantes : R⁶, R⁷, R⁸ et R⁹ sont des atomes d'hydrogène ; dans Z, n1, n2 et n3 sont égaux à 0.

4. Procédé selon la revendication 3, caractérisé par la séparation d'énantiomères, le radical A de la formule I étant le groupe éthyle.

5. Procédé selon l'une des revendications 1 à 4, caractérisé en ce que le solvant contient du tris(3,5-diméthylphénylcarbamate) de cellulose.

6. Procédé selon l'une des revendications 1 à 5, caractérisé en ce que l'éluant utilisé est un alcool en C₁ à C₅.

7. Procédé selon l'une des revendications 1 à 5, caractérisé en ce qu'on utilise en tant qu'éluant un mélange contenant un alcool en C₁ à C₅ et un hydrocarbure en C₅ à C₁₀.

8. Procédé selon l'une des revendications 1 à 7, caractérisé en ce que le procédé est mis en oeuvre d'une manière discontinue.

9. Procédé selon l'une des revendications 1 à 7, caractérisé en ce que le procédé est mis en oeuvre d'une manière continue par le procédé en lit mobile simulé SMB.
